# EUROPÄISCHE PATENTSCHRIFT

(11) **EP 1 333 867 B1**
(45) Veröffentlichungstag und Bekanntmachung des Hinweises auf die Patenterteilung: **19.03.2008**
(21) Anmeldenummer: 01990413.5
(22) Anmeldetag: 14.11.2001
(51) Int. Cl.: A61L 2/28, G01K 13/00

(54) **MESSVORRICHTUNG ZUR ERFASSUNG VON TEMPERATURABHÄNGIGEN MESSWERTEN**
MEASURING DEVICE FOR DETECTING TEMPERATURE DEPENDENT MEASURED VALUES
DISPOSITIF DE MESURE POUR LA DETECTION DE VALEURS DE MESURE FONCTION DE LA TEMPERATURE

(30) Priorität: 15.11.2000 DE 20019360 U
(43) Veröffentlichungstag der Anmeldung: 13.08.2003
(73) Patentinhaber: ebro Electronic GmbH & Co. KG, 85055 Ingolstadt (DE)
(72) Erfinder: BOSCH, Albert, NL-5935 CE Steyl (NL)
(74) Vertreter: Bergmeier, Werner
(86) Internationale Anmeldenummer: PCT/EP2001/013178
(87) Internationale Veröffentlichungsnummer: WO 2002/040066

(56) Entgegenhaltungen:
- EP-A- 0 982 039
- EP-A- 1 112 751
- WO-A-95/32742
- DE-U- 9 319 369
- US-A- 5 491 092

## Beschreibung

Die Erfindung betrifft eine Messvorrichtung zur Erfassung von temperaturabhängigen Messwerten oder temperaturabhängigen Kombinationen physikalischer Einflussfaktoren, insbesondere zur Messung von Temperaturen in Sterilisatoren, mit zumindest einer Messeinrichtung, wobei die Wärmekapazität der Messeinrichtung durch ein mit der Messzone der Messeinrichtung zumindest bereichsweise in wärmeleitendem Kontakt stehendes, zusätzliches wärmeaufnehmendes Material erhöht wird

Wäschestücke, Operationswerkzeuge oder sonstige zu sterilisierende Güter werden in Sterilisationskammern sterilisiert. Bei der Dampfsterilisation wird Dampf zu Beginn des Sterilisationsprozesses in die Sterilisationskammer geleitet. Da Dampf sich nicht mit der noch in der Sterilisationskammer befindlichen Luft vermischt, muss in Abständen das Einströmen des Dampfes unterbrochen und über eine Vakuumpumpe die noch in der Sterilisationskammer befindliche Luft bzw. nichtkondensierbare Gase, die im Dampf enthalten sein können, abgezogen werden. Dieser Vorgang wird mehrfach wiederholt.

Sobald sich in der Sterilisationskammer keine Luft mehr befindet, wird über einen Zeitraum von etwa drei Minuten bei 2 Bar der 134°C heiße Sattdampf in die Sterilisationskammer geführt, wobei der Sattdampf auf dem eigentlichen zu sterilisierenden Gut kondensiert und dabei Kondensationswärme frei wird. Hierdurch findet die eigentliche Sterilisation statt.

Im Anschluss daran wird die Sterilisationskammer geöffnet, so dass der Dampf aus dieser austreten kann. Gleichzeitig wird ein Vakuum gezogen, so dass der Siedepunkt des Wassers auf etwa 20°C abgesenkt wird. Nach etwa 10 Minuten sind die zu sterilisierenden Güter trocken, und der Sterilisationsprozess ist beendet.

Zur Kontrolle eines einwandfreien Sterilisationsprozesses wird u.a. der Druck gemessen. Bei Bedarf werden noch sogenannte chemische Indikatoren zwischen die zu sterilisierenden Güter gelegt, die bei Vorliegen bestimmter Zeit und Temperaturparameter sowie bei einem gewissen Feuchtigkeitsgrad farblich umschlagen. Sofern überprüft werden soll, ob die Vakuumpumpen ein hinreichendes Vakuum erzeugen, werden die chemischen Indikatoren in einen Hohlkörper mit einer Öffnung, an der eine Barriere wie z. B. ein angeformter Kanal angeordnet sein kann, der seinerseits zwischen den zu sterilisierenden Gütern platziert wird. Ein farblicher Umschlag ist nur dann möglich, wenn der Dampf in den Hohlkörper eintreten kann, was wiederum ein zuvor erzeugtes Vakuum in dem Hohlkörper unumgänglich macht.

Gleichzeitig muss aber auch fortwährend die Temperatur überwacht werden, wozu im Ausguss der Sterilisationskammer ein Thermoelement vorgesehen ist. Dieser gemessene Wert erlaubt aber keine genauen Rückschlüsse darauf, ob auch innerhalb der zu sterilisierenden Güter wie z.B. gestapelte Bekleidungsstücke diese Temperatur herrscht. Ferner ist insbesondere bei Thermoelementen nicht feststellbar, ob die gemessene Temperatur durch Strahlungswärme oder aber - was essenziell wichtig ist - durch die Kondensationswärme entstanden ist. Denn nur durch die frei werdende Kondensationswärme findet eine hinreichende Sterilisation statt.

In der EP 1 112 751 A1 wird ein Verfahren und eine Vorrichtung zur Messung der Konzentration von Sterilisationsgasen bzw. -dämpfen innerhalb einer Sterilisationskammer vorgeschlagen. Hierfür wird eine chemische Substanz über einen Träger mit einer Temperaturmesssonde verbunden. Aus der Temperaturerhöhung, die durch die exotherme Reaktion zwischen der chemischen Substanz und einem Sterilisationsgas bzw. -dampf entsteht, wird schließlich die Gas- bzw. Dampfkonzentration ermittelt. Hierdurch sind jedoch keinerlei Rückschlüsse auf das eigentliche Sterilisationsergebnis möglich.

Die WO 95/32742 A1 beschreibt eine elektronische Testpackung zur Überprüfung der Effektivität eines Sterilisationsvorganges. Diese wird in einer Sterilisationskamme platziert und zeichnet darin physikalische Messgrößen an zwei voneinander beabstandeten Stellen auf, wobei zwischen beiden Stellen ein poröses Material eingebracht ist, welches das Sterilisationsmedium durchdringen muss, um zur zweiten Messstelle zu gelangen. Aus den aufgezeichneten Messwerten werden schließlich Rückschlüsse gezogen, inwieweit das Sterilisationsmedium auch das zu sterilisierende Produkt vollständig durchdrungen hat. Die Vorrichtung weist jedoch einen relativ komplizierten Aufbau auf und verringert hierdurch auch in nicht zu vernachlässigender Weise das Volumen der Sterilisationskammer.

Aufgabe der Erfindung ist es, eine Messvorrichtung der bekannten Art so zu verbessern, dass die gemessene Temperatur auch tatsächlich durch die freigewordene Kondensationswärme entstanden ist.

Diese Aufgabe wird dadurch gelöst, dass zur Reduzierung der Auswirkung von kurzfristigen Temperatureinflüssen auf den Messwert das zusätzliche wärmeaufnehmende Material eine höhere Wärmekapazität aufweist als die Messzone. Hierdurch ersetzt die höhere Wärmekapazität der Gesamtheit, bestehend aus Messeinrichtung und zusätzlichem Material die niedrigere Wärmekapazität der Messeinrichtung in Alleinstellung, so dass eine größere Wärmemenge aufgenommen werden kann, d. h. eine längere Beeinflussung mit einer bestimmten Temperaturdifferenz erforderlich ist, um eine Messwertveränderung zu bewirken. Aufgrund seiner guten Wärmeleitfähigkeit und der zusätzlichen Wärmekapazität nimmt das zusätzliche Material die Wärme auf und gewährleistet somit, dass sich bei geringeren Energiezuführungen z. B. durch Strahlungswärme die Temperatur der Messeinrichtung wenig verändert.

Dabei kann die zusätzliche Wärmekapazität aus einer größeren Menge eines zusätzlichen Materials mit einer geringeren spezifischen Wärmekapazität oder aus einer geringeren Menge eines zusätzlichen Materials mit einer entsprechend höheren spezifischen Wärmekapazität resultieren.

Wird z. B. die Messeinrichtung lediglich durch die Strahlungswärme oder nicht kondensierbare Gase kurzzeitig erwärmt, so wird die Wärme direkt von dem Material, das viel Wärme aufnehmen kann, aufgenommen. Dieses wird aber infolge der höheren Masse nicht so stark erwärmt. Infolgedessen kühlt sich die Messeinrichtung wieder ab. Erst wenn durch die Kondensation des Sattdampfes erhebliche Wärmemengen frei werden, so dass nicht nur die Messeinrichtung sondern auch das Material erwärmt werden, wird sichergestellt, dass der zu diesem Zeitpunkt gemessene Temperaturanstieg durch die freigewordene Kondensationswärme verursacht wird. Mit der Messeinrichtung kann beispielsweise direkt die Temperatur gemessen werden.

Das Material kann beispielsweise die Form eines Quaders aufweisen und kann seitlich und/oder stirnseitig an der Messeinrichtung angeordnet sein.

Bei einer anderen Ausführungsform kann das Material die Messeinrichtung zumindest bereichsweise umgeben.

Vorteilhafterweise kann das Material als eine vorzugsweise endseitig geschlossene Hülse ausgebildet sein, die auf die Messeinrichtung im Bereich der Messzone aufgesteckt wird.

Das Material kann dabei kupfer-, aluminium- und/oder bleihaltig sein. Es können aber auch andere Materialien verwendet werden, wie z. B. Metalle vorzugsweise Edelstahl oder Metalllegierungen oder Kunststoffe.

Bei einer Ausführungsform kann die Messeinrichtung als ein chemischer Indikator ausgebildet sein. Dies erlaubt das Messen von temperaturabhängigen Messwerten oder temperaturabhängigen Kombinationen physikalischer Einflussfaktoren, die einen Rückschluss auf die in der Sterilisationskammer herrschenden Temperaturen erlauben. Da die chemischen Indikatoren u.a. zeit- und temperaturabhängig sind, ist bei einem solchen Ausführungsbeispiel auf jeden Fall gewährleistet, dass ein eventueller optischer Farbumschlag bei einer genau vorgegebenen Temperatur erfolgt ist. Da das Material mit dem chemischen Indikator in Kontakt ist, ist sichergestellt, dass die für den Farbumschlag erforderliche Temperatur durch die freigewordene Kondensationswärme verursacht worden ist.

Die Messeinrichtung kann ein Thermoelement sein. Es sind aber durchaus auch andere elektrisch arbeitende Messeinrichtungen, wie z.B. Infrarot-Thermometer möglich.

Die Messeinrichtung kann mit einer Messwertspeicher- und Energieversorgungseinrichtung verbunden sein, so dass es keiner Anschlüsse der Messvorrichtung selbst mehr mit einer z.B. außerhalb der Sterilisationskammer befindlichen Auswerteeinrichtung bedarf.

Zweckmäßigerweise kann eine Schnittstelle insbesondere für den Datentransfer vorgesehen sein. Zu Beginn des Sterilisationsprozesses wird die Messvorrichtung zwischen das zu sterilisierende Gut gelegt. Nach Beendigung des Sterilisationsprozesses wird die Messvorrichtung aus der Sterilisationskammer genommen und die auf der Messwertspeichereinrichtung hinterlegten Daten über die Schnittstelle beispielsweise auf einen Computer übertragen.

Zweckmäßigerweise kann zusätzlich eine Zählvorrichtung für die Anzahl der bereits erfolgten Messungen vorgesehen sein. Dies ist insbesondere dann erforderlich, wenn der Gesetzgeber Wartungen der Messvorrichtung nach einer bestimmten Anzahl von Messungen vorschreibt. Sofern die gesetzlich vorgeschriebene Anzahl der Messungen überschritten wird, kann beispielsweise ein optisches oder akustisches Warnsignal ausgegeben werden.

Zumindest die Messeinrichtung kann in einem zumindest eine Öffnung aufweisenden Hohlkörper angeordnet sein.

Dabei kann an die Öffnung ein rohr- bzw. schlauchförmig ausgebildeter Kanal angeformt sein. Ein solcher Hohlkörper mit einem angeformten Kanal simuliert Operationswerkzeuge mit vergleichbaren rohrförmigen Bauteilen, so dass auf diese Weise überprüft werden kann, ob während des Sterilisationsprozesses ein hinreichender Unterdruck erzeugt wird. Zunächst wird bei der Erzeugung des Vakuums in der Sterilisationskammer u.a. auch die Luft aus dem Kanal und dem Hohlkörper gezogen. Erst wenn in dem Hohlkörper ein Vakuum vorliegt, kann der Dampf durch den so erzeugten Unterdruck durch den Kanal bis in den Hohlkörper hineingezogen werden. Sofern sich in dem Hohlkörper ein chemischer Indikator befindet, kann so die Betriebsweise der Sterilisationskammer überprüft werden.

Im Folgenden wird ein in der Zeichnung dargestelltes Ausführungsbeispiel der Erfindung erläutert. Es zeigen:
- Fig. 1: eine Seitenansicht auf eine erfindungsgemäße Messeinrichtung mit seitlich angeordnetem Material,
- Fig. 2: eine Seitenansicht auf eine Messeinrichtung, bei der das Material als eine endseitig aufgesteckte Hülse ausgebildet ist und
- Fig. 3: einen teilweisen Schnitt durch einen Hohlkörper mit einem angeformten Kanal und darin befindlicher Messeinrichtung.

In allen Figuren werden für gleiche bzw. gleichartige Bauteile übereinstimmende Bezugszeichen verwendet.

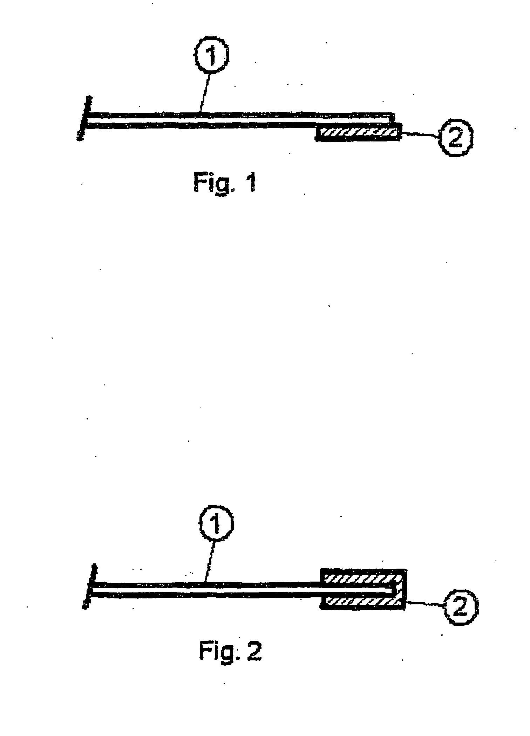

Fig. 1 zeigt eine Messeinrichtung 1, an der endseitig ein mit der Messeinrichtung 1 in wärmeleitfähigem Kontakt stehendes Material 2 angeordnet ist. Das Material 2 ist in dem dargestellten Ausführungsbeispiel seitlich an der Messeinrichtung 1 angeordnet.

Das Material 2 ist in seiner Eigenschaft und in seiner Masse so auf die Messeinrichtung 1 abgestimmt, dass die Gesamtheit, bestehend aus Messeinrichtung 1 und zusätzlichem Material 2 eine höhere Wärmekapazität aufweist, so dass eine größere Wärmemenge, d. h. eine längere Beeinflussung mit einer bestimmten Temperaturdifferenz erforderlich ist, um eine Messwertveränderung zu bewirken. Wird die Messeinrichtung 1 z. B. durch Strahlungswärme kurzzeitig erwärmt, wird die Wärme direkt in das Material 2 weitergeleitet. Aufgrund der größeren Masse des Materials 2 erwärmt sich, dieses nicht so stark. Aufgrund des Wärmeentzuges durch das Material 2 kühlt sich die Messeinrichtung 1 entsprechend ab, so dass die durch die Strahlungswärme kurzzeitig auftretende erhöhte Temperatur in der Messeinrichtung 1 nicht gemessen wird. Wird hingegen Kondensationswärme frei, die sich durch die Kondensation des Sattdampfes bildet, so erwärmt sich auch das Material 2 entsprechend. Die so gemessene Temperatur entspricht dann genau dem Temperaturanstieg, der durch die Kondensationswärme verursacht worden ist.

Die Messeinrichtung 1 ist in dem dargestellten Ausführungsbeispiel als Thermoelement ausgebildet, das mit einer nicht dargestellten Anzeige- bzw. Auswerteeinrichtung verbunden ist. Es sind aber auch andere Messeinrichtungen wie z.B. Quecksilberthermometer oder Infrarot-Thermometer denkbar.

Bei dem in Fig. 2 dargestellten Ausführungsbeispiel ist das Material 2 als eine endseitig geschlossene Hülse ausgebildet, die endseitig auf die Messeinrichtung 1 aufgeschoben wird.

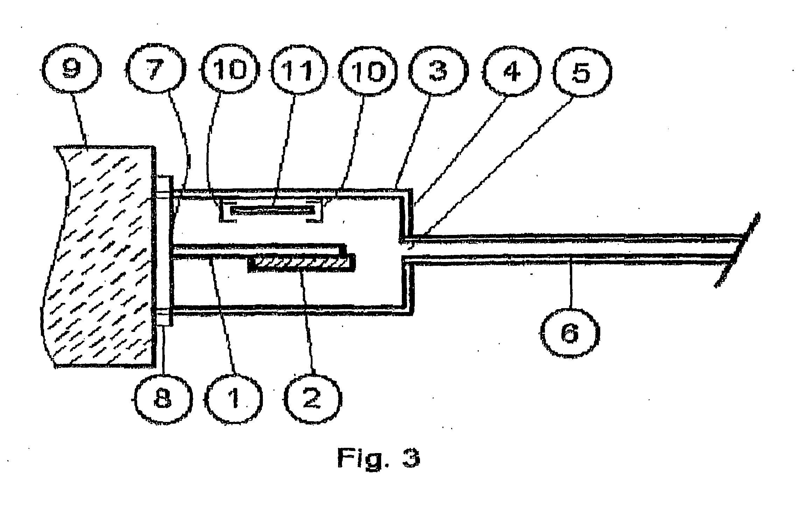

In Fig. 3 ist die Messeinrichtung 1 mit dein endseitig angeordneten Material 2 in einem Hohlkörper 3 angeordnet. Der Hohlkörper 3 weist in einer Stirnwand 4 eine Öffnung 5 auf, wobei an die Öffnung 5 ein schlauchförmiger Kanal 6 angeformt ist. Die andere Stirnwand 7 ist als Schraubverschluss 8, an den in dem dargestellten Ausführungsbeispiel eine Messwertspeicher- und Energieversorgungseinrichtung 9 angeordnet ist, ausgebildet. Von der Messwertspeicher- und Energieversorgungseinrichtung 9 ragt durch den Schraubverschluss 8 die Messeinrichtung 1 mit dem in wärmeleitendem Kontakt stehenden Material 2 in den Hohlkörper 3.

Die Messwertspeicher- und Energieversorgungseinrichtung 9 ist hitzebeständig und kann sich insoweit während des Sterilisationsprozesses im Inneren der Sterilisationskammer befinden. Im Anschluss an den Sterilisationsprozess können die in der Messwertspeicher- und Energieversorgungseinrichtung 9 hinterlegten Daten beispielsweise über eine Schnittstelle auf eine Auswertevorrichtung, die sich außerhalb der Sterilisationskammer befindet, übertragen werden. Es ist aber auch durchaus denkbar, dass die gemessenen Daten per Funk an einen außerhalb der Sterilisationskammer befindlichen Empfänger gesandt werden.

Zum Öffnen des Hohlkörpers 3 wird der Schraubverschluss 8 entfernt, und die Messeinrichtung 1 ist so zugänglich. Im Anschluss daran wird der Hohlkörper 3 mittels des Schraubverschlusses 8 wieder verschlossen.

In dem Hohlkörper 3 ist ferner eine Haltevorrichtung 10 vorgesehen, die z.B. zur Halterung eines chemischen Indikators 11 dient. Dieser chemische Indikator 11 wechselt bei einem bestimmten Feuchtigkeitsgehalt, wobei auch gleichzeitig bestimmte Temperatur- und Zeitparameter vorliegen müssen, seine Farbe.

Der Hohlkörper 3 mit dem angeformten Kanal 6 dient zur Simulation beispielsweise eines Operationswerkzeuges mit einem länglichen rohrförmigen Aufbau, das in einer Sterilisationskammer sterilisiert werden soll. Durch die Anordnung von den Hohlkörpern 3 und dem Kanal 6 wird kontrolliert, ob die Vakuumpumpen der Sterilisationskammer ein hinreichendes Vakuum erzeugen, da nur bei einem ausreichend erzeugten Vakuum Dampf durch den Kanal 6 in das innere des Hohlkörpers 3 eintreffen und für einen Farbumschlag des chemischen Indikators 11 sorgen kann.

Die Messeinrichtung 1 kann aber auch beispielsweise über Kabel mit einer vorzugsweise außerhalb der Sterilisationskammer angeordneten Auswerte- und Anzeigevorrichtung verbunden sein. In diesem Fall bedürfte es auch nicht der Messwertspeicher- und Energieversorgungseinrichtung 9.

## Patentansprüche

1. Messvorrichtung zur Erfassung von temperaturabhängigen Messwerten oder temperaturabhängigen Kombinationen physikalischer Einflussfaktoren, insbesondere zur Messung von Temperaturen in Sterilisatoren, mit zumindest einer Messeinrichtung (1), wobei die Wärmekapazität der Messeinrichtung (1) durch ein mit der Messzone der Messeinrichtung (1) zumindest bereichsweise in wärmeleitendem Kontakt stehendes, zusätzliches wärmeaufnehmendes Material (2) erhöht wird, **dadurch gekennzeichnet, dass** zur Reduzierung der Auswirkung von kurzfristigen Temperatureinflüssen auf den Messwert das zusätzliche wärmeaufnehmende Material (2) eine höhere Wärmekapazität aufweist als die Messzone.

2. Messvorrichtung nach Anspruch 1, **dadurch gekennzeichnet, dass** das Material (2) seitlich an der Messeinrichtung (1) angeordnet ist.

3. Messvorrichtung nach Anspruch 1 oder 2, **dadurch gekennzeichnet, dass** das Material (2) stirnseitig an der Messeinrichtung (1) angeordnet ist.

4. Messvorrichtung nach einem der Ansprüche 1 bis 3, **dadurch gekennzeichnet, dass** das Material (2) die Messeinrichtung (1) zumindest bereichsweise umgibt.

5. Messvorrichtung nach Anspruch 4, **dadurch gekennzeichnet, dass** das Material (2) als eine vorzugsweise endseitig geschlossene Hülse ausgebildet ist.

6. Messvorrichtung nach einem der Ansprüche 1 bis 5, **dadurch gekennzeichnet, dass** das Material (2) kupferhaltig ist.

7. Messvorrichtung nach einem der Ansprüche 1 bis 6, **dadurch gekennzeichnet, dass** das Material (2) aluminiumhaltig ist.

8. Messvorrichtung nach einem der Ansprüche 1 bis 7, **dadurch gekennzeichnet, dass** das Material (2) bleihaltig ist.

9. Messvorrichtung nach einem der Ansprüche 1 bis 8, **dadurch gekennzeichnet, dass** das Material (2) ein Metall, vorzugsweise Edelstahl ist.

10. Messvorrichtung nach einem der Ansprüche 1 bis 9, **dadurch gekennzeichnet, dass** die Messeinrichtung (1) als ein chemischer Indikator (11) ausgebildet ist.

11. Messvorrichtung nach einem der Ansprüche 1 bis 9, **dadurch gekennzeichnet, dass** die Messeinrichtung (1) ein Thermoelement ist.

12. Messvorrichtung nach Anspruch 11, **dadurch gekennzeichnet, dass** die Messeinrichtung (1) mit einer Messwertspeicher- und Energieversorgungseinrichtung (9) verbunden ist.

13. Messvorrichtung nach Anspruch 12, **dadurch gekennzeichnet, dass** eine Schnittstelle insbesondere für den Datentransfer vorgesehen ist.

14. Messvorrichtung nach einem der Ansprüche 11 bis 13, **dadurch gekennzeichnet, dass** zusätzlich eine Zählvorrichtung für die Anzahl der bereits erfolgten Messungen vorgesehen ist.

15. Messvorrichtung nach einem der Ansprüche 1 bis 14, **dadurch gekennzeichnet, dass** zumindest die Messeinrichtung (1) in einem zumindest eine Öffnung (5) aufweisenden Hohlkörper (3) angeordnet ist.

16. Messvorrichtung nach Anspruch 15, **dadurch gekennzeichnet, dass** an die Öffnung (5) ein rohr- bzw. schlauchförmig ausgebildeter Kanal (6) angeformt ist.

## Claims

1. Measuring device to detect temperature-dependent measured values or temperature-dependent combinations of physical influencing factors, in particular for measuring temperatures in sterilizers, with at least one measuring device (1), the heat capacity of the measuring device (1) being increased by an additional heat-absorbing material (2) which is in heat-conducting contact with the measure zone of the measuring device (1) at least in certain areas, **characterized in that** the additional heat-absorbing material (2) shows a higher heat capacity than the measure zone, in order to reduce the effect of short-term temperature influences on the measured value.

2. Measuring device according to claim 1, **characterized in that** the material (2) is placed on the side of the measuring device (1).

3. Measuring device according to claim 1 or 2, **characterized in that** the material (2) is placed in front of the measuring device (1).

4. Measuring device according to one of the claims 1 to 3, **characterized in that** the material (2) surrounds the measuring device (1) at least in certain areas.

5. Measuring device according to claim 4, **characterized in that** the material (2) is in the form of a shell preferably closed at one end.

6. Measuring device according to one of the claims 1 to 5, **characterized in that** the material (2) contains copper.

7. Measuring device according to one of the claims 1 to 6, **characterized in that** the material (2) contains aluminum.

8. Measuring device according to one of the claims 1 to 7, **characterized in that** the material (2) contains lead.

9. Measuring device according to one of the claims 1 to 8, **characterized in that** the material (2) is a metal, preferably stainless steel.

10. Measuring device according to one of the claims 1 to 9, **characterized in that** the measuring device (1) is in the form of a chemical indicator (11).

11. Measuring device according to one of the claims 1 to 9, **characterized in that** the measuring device (1) is a thermoelement.

12. Measuring device according to claim 11, **characterized in that** the measuring device (1) is connected to a measured value storage device and power supply (9).

13. Measuring device according to claim 12, **characterized in that** an interface, in particular for data transfer, is provided.

14. Measuring device according to one of the claims 11 to 13, **characterized in that** in addition a counter for counting the number of the completed measurements is provided.

15. Measuring device according to one of the claims 1 to 14, **characterized in that** at least the measuring device (1) is placed in a hollow piece (3) having at least one opening (5).

16. Measuring device according to claim 15, **characterized in that** a canal (6) in the form of a tube or hose is fitted to the opening (5).

## Revendications

1. Dispositif de mesure pour l'acquisition de valeurs de mesure variables avec la température ou de combinaisons variables avec la température de facteurs d'influence physiques, particulièrement pour la mesure des températures dans des stérilisateurs, avec au moins un système de mesure (1), sachant que la capacité thermique du système de mesure (1) est augmentée par une matière endothermique (2) supplémentaire en contact thermoconducteur avec au moins une partie de la zone de mesure du système de mesure (1), **caractérisé en ce que** pour la réduction de l'effet des influences de températures de courte durée sur la valeur de mesure, la matière endothermique supplémentaire (2) présente une capacité thermique supérieure à celle de la zone de mesure.

2. Dispositif de mesure selon la revendication 1, **caractérisé en ce que** la matière (2) est disposée latéralement à côté du système de mesure (1).

3. Dispositif de mesure selon l'une quelconque des revendications 1 ou 2, **caractérisé en ce que** la matière (2) est disposée à la face du système de mesure (1).

4. Dispositif de mesure selon l'une quelconque des revendications 1 à 3, **caractérisé en ce que** la matière (2) entoure le système de mesure (1) au moins en certaines zones.

5. Dispositif de mesure selon la revendication 4, **caractérisé en ce que** la matière (2) se présente sous la forme d'une douille fermée de préférence à son extrémité.

6. Dispositif de mesure selon l'une quelconque des revendications 1 à 5, **caractérisé en ce que** la matière (2) contient du cuivre.

7. Dispositif de mesure selon l'une quelconque des revendications 1 à 6, **caractérisé en ce que** la matière (2) contient de l'aluminium.

8. Dispositif de mesure selon l'une quelconque des revendications 1 à 7, **caractérisé en ce que** la matière (2) contient du plomb.

9. Dispositif de mesure selon l'une quelconque des revendications 1 à 8, **caractérisé en ce que** la matière (2) est un métal, de préférence un acier surfin.

10. Dispositif de mesure selon l'une quelconque des revendications 1 à 9, **caractérisé en ce que** le système de mesure (1) se présente sous la forme d'un indicateur chimique (11).

11. Dispositif de mesure selon l'une quelconque des revendications 1 à 9, **caractérisé en ce que** la matière (1) est un thermocouple.

12. Dispositif de mesure selon la revendication 11, **caractérisé en ce que** le système de mesure (1) est relié à un dispositif de mémorisation des valeurs de mesure et d'alimentation en énergie (9).

13. Dispositif de mesure selon la revendication 12, **caractérisé en ce qu'il** est prévu une interface, particulièrement pour le transfert des données.

14. Dispositif de mesure selon l'une quelconque des revendications 11 à 13, **caractérisé en ce qu'il** est prévu de surcroit un dispositif de comptage pour le nombre de mesures déjà effectuées.

15. Dispositif de mesure selon l'une quelconque des revendications 1 à 14, **caractérisé en ce que** le système de mesure (1) au moins est disposé dans un corps creux (3) comportant au moins une ouverture (5).

16. Dispositif de mesure selon la revendication 15, **caractérisé en ce qu'un** canal (6) se présentant sous la forme d'un tube ou d'un tuyau est disposé à l'ouverture (5).
